(19) Europäisches Patentamt / European Patent Office / Office européen des brevets

(11) **EP 4 400 839 A1**

(12) **EUROPEAN PATENT APPLICATION**
published in accordance with Art. 153(4) EPC

(43) Date of publication:
**17.07.2024 Bulletin 2024/29**

(21) Application number: **21955583.6**

(22) Date of filing: **06.09.2021**

(51) International Patent Classification (IPC):
*G01N 33/48* *(2006.01)*

(52) Cooperative Patent Classification (CPC):
**G01N 33/48**

(86) International application number:
**PCT/CN2021/116782**

(87) International publication number:
**WO 2023/029052 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Oxford University (Suzhou) Science & Technology Co., Ltd.**
**Suzhou, Jiangsu 215123 (CN)**

(72) Inventors:
- **YEUNG, Danny Sheng Wu**
  **Hong Kong 999077 (HK)**
- **MA, Wu-Po**
  **Hong Kong 999077 (HK)**
- **WU, Shu**
  **Hong Kong 999077 (HK)**
- **WONG, Yung Ho Peter**
  **Hong Kong 999077 (HK)**

- **SUNDARAM, Senthil Kumar**
  **Hong Kong 999077 (HK)**
- **YING, Dingge**
  **Hong Kong 999077 (HK)**
- **CHU, Hoi Ting Janette**
  **Hong Kong 999077 (HK)**
- **TAN, Kevin Clark**
  **Hong Kong 999077 (HK)**
- **HSU, Chia-Chen**
  **Hong Kong 999077 (HK)**
- **DOMINGUES, Allysson Ferreira**
  **Hong Kong 999077 (HK)**
- **MAUND, Alex**
  **Hong Kong 999077 (HK)**
- **CHUNG-HOW, William**
  **Hong Kong 999077 (HK)**

(74) Representative: **Patentanwaltskanzlei WILHELM & BECK**
**Prinzenstraße 13**
**80639 München (DE)**

(54) **REAGENT REACTION DETERMINATION METHOD AND APPARATUS, STORAGE MEDIUM AND TEST TUBE**

(57)    A reagent reaction determination method and device applied to a test tube, a computer-readable storage medium and a test tube, wherein, the reagent reaction determination method comprises: outputting a brightness control signal to the light-incident component to control the light-incident component to emit the RGB light, and to adjust the brightness control signal according to a preset step length, so as to adjust the brightness of the RGB light; detecting brightness variations of the light derived by the light-emergent component to generate an electrical signal detection value; perform an interpolation calculation on the present brightness control signal to obtain an estimated value of the brightness control signal when the electrical signal detection value is less than a preset threshold, and determining a reaction result in the reagent reaction chamber according to the estimated value of the brightness control signal. Therefore, the reagent reaction determination device applied to a test tube in the embodiment is capable of improving the accuracy of a reagent reaction determination, while simplifying the determination steps and reducing the determination time, which greatly improves the user experience.

EP 4 400 839 A1

**(Cont. next page)**

S10 — Output a brightness control signal to the light-incident component to control the light-incident component to emit the RGB light, and to adjust the brightness control signal according to a preset step length, so as to adjust the brightness of the RGB light

S20 — Detect brightness variations of the light derived by the light-emergent component, so as to generate an electrical signal detection value

S30 — When the electrical signal detection value is less than a preset threshold, performing an interpolation calculation on a present brightness control signal to obtain an estimated value of the brightness control signal, and determining a reaction result in the reagent reaction chamber according to the estimated value of the brightness control signal

Fig. 4

**Description**

**Cross-reference to Related Applications**

This application claims the priority to PCT/CN2021/116782, entitled "Reagent reaction determination method and device, storage medium and test tube" and filed on September 6, 2021, the entirety of which is incorporated herein by reference.

**Field of the Invention**

The present disclosure relates to the technical field of reagent detection, in particular to a reagent reaction determination method applied to a test tube, a computer-readable storage medium, a test tube and a reagent reaction determination device applied to a test tube.

**Background of the Invention**

With the spread of COVID-19 virus, people's daily epidemic prevention is particularly important, and nucleic acid detection is currently a more accurate way to screen infected people, and can be effectively applied to epidemic prevention work.

In related technologies, detecting results are usually obtained by collecting nucleic acid concentratedly and then transported to a laboratory for testing by laboratory instruments. The foregoing detection method has the following drawbacks: 1. People being collected often tend to bunch up during the collection process, and if there are infected people among them, it is very easy for infection to occur, which is not conducive to the epidemic prevention. 2. Collected nucleic acid samples need to be transported to the laboratory for testing, which is fraught with uncertainties and hazards, and weather and road conditions can affect the transportation of the samples. 3. Long testing time and normal test results with cumbersome steps lead to poor user experience.

**Summary of the Invention**

The present disclosure is intended to solve at least one of the technical problems in the related art to some extent. Therefore, a first object of the present disclosure is to propose a reagent reaction determination method applied to a test tube, which is capable of improving the accuracy of a reagent reaction determination, while simplifying the determination steps and reducing the determination time, which greatly improves the user experience.

A second object of the present disclosure is to propose a computer-readable storage medium.

A third object of the present disclosure is to propose a test tube.

A fourth object of the present disclosure is to propose a reagent reaction determination device applied to a test tube.

For the above purposes, in a first aspect of the present disclosure, some embodiments provide a reagent reaction determination method applied to a test tube, wherein the test tube is internally provided with a sampling chamber suitable for accommodating a sampling tube, at least one reagent reaction chamber is formed in the bottom panel of the sampling chamber. And each of the reagent reaction chamber is correspondingly provided with a light-incident component and a light-emergent component, the light-incident component guides RGB light to the reagent reaction chamber, and the light-emergent component guides a light passing through the reagent reaction chamber. The method comprises: outputting a brightness control signal to the light-incident component, so as to control the light-incident component to emit the RGB light, and adjusting the brightness control signal according to a preset step length, so as to adjust the brightness of the RGB light; detecting brightness variations of the light derived by the light-emergent component, so as to generate an electrical signal detection value; when the electrical signal detection value is less than a preset threshold, performing an interpolation calculation on a present brightness control signal to obtain an estimated value of the brightness control signal, and determining a reaction result in the reagent reaction chamber according to the estimated value of the brightness control signal.

In some embodiments of the present disclosure, the test tube is internally provided with a sampling chamber suitable for accommodating a sampling tube, at least one reagent reaction chamber is formed in the bottom panel of the sampling chamber. And each of the reagent reaction chamber is correspondingly provided with a light-incident component and a light-emergent component, the light-incident component guides RGB light to the reagent reaction chamber and the light-emergent component guides the RGB light passing through the reagent reaction chamber. The reagent reaction determination method applied to a test tube in this embodiment, firstly output a brightness control signal to the light-incident component, so as to control the light-incident component to emit the RGB light, and adjust the brightness control signal according to a preset step length, so as to adjust the brightness of the RGB light; then detect brightness variations of the light derived by the light-emergent component, so as to generate an electrical signal detection value. When the electrical signal detection value is less than a preset threshold, perform an interpolation calculation on a present brightness control signal to obtain an estimated value of the brightness control signal, and finally determine a reaction result in the reagent reaction chamber according to the estimated value of the brightness control signal. As a result, the reagent reaction determination method applied to a test tube in this embodiment is capable of improving the accuracy of a reagent reaction determination, while simplifying the determination steps and reducing the determination time, which greatly improves the user experience.

In some embodiments of the present disclosure, when the electrical signal detection value is greater than or equal to the preset threshold, return to continue adjusting the brightness control signal until the electrical signal detection value is less than the preset threshold.

In some embodiments of the present disclosure, performing the interpolation calculation on the present brightness control signal comprises:
determining the estimated value of the brightness control signal according to the preset step length, the preset threshold, and a present electrical signal detection value, a present value of the brightness control signal and a last electrical signal detection value when the electrical signal detection value is less than the preset threshold.

In some embodiments of the present disclosure, the estimated value of the brightness control signal is calculated according to the following formula:
$Y=Y1+a*[(X0-X1)/(X-X1)]$, wherein Y is the estimated value of the brightness control signal, Y1 is the present value of the brightness control signal, a is the preset step length, X0 is the preset threshold, X1 is the last electrical signal detection value, X is the present electrical signal detection value.

In some embodiments of the present disclosure, when obtaining the estimated value of the brightness control signal, the method further comprises: determining a reagent reaction time, and when the reagent reaction time exceeds a first preset time, performing a standardized processing on the estimated value of the brightness control signal to obtain a standardized processing value, so as to determine the reaction result in the reagent reaction chamber according to the standardized processing value.

In some embodiments of the present disclosure, performing the standardized processing on the estimated value of the brightness control signal comprises:
determining the sum of the estimated value of the present brightness control signal and the estimated value of the brightness control signal within an effective reaction time, calculating an average value of the brightness control signal within the effective reaction time, and dividing the estimated value of the present brightness control signal by the average value of the brightness control signal within the effective reaction time, so as to obtain the standardized processing value.

In some embodiments of the present disclosure, the standardized processing value comprises a processing value of the brightness control signal corresponding to a blue light and a processing value of the brightness control signal corresponding to a green light in the RGB light, or a processing value of the brightness control signal corresponding to a red light and a processing value of the brightness control signal corresponding to a green light in the RGB light, wherein, determining the reaction result in the reagent reaction chamber according to the standardized processing value, comprising: 1.when the difference between the processing value of the brightness control signal corresponding to a blue light and the processing value of the brightness control signal corresponding to a green light is greater than a first preset difference, or the processing value of the brightness control signal corresponding to a red light and the processing value of the brightness control signal corresponding to a green light is greater than a second preset difference, determining that the reaction result in the reagent reaction chamber is positive. 2. when the difference between the processing value of the brightness control signal corresponding to a blue light and the processing value of the brightness control signal corresponding to a green light is less than the first preset difference or the processing value of the brightness control signal corresponding to a red light and the processing value of the brightness control signal corresponding to a green light is less than the second preset difference, determining that the reaction result in the reagent reaction chamber is negative.

In some embodiments of the present disclosure, there are a plurality of reagent reaction chambers, and one of the reagent reaction chambers is a standard control chamber, and the method further comprises: 1.when the difference between the processing value of the brightness control signal corresponding to a blue light and the processing value of the brightness control signal corresponding to a green light is greater than the first preset difference, or the processing value of the brightness control signal corresponding to a red light and the processing value of the brightness control signal corresponding to a green light is greater than the second preset difference, if the reagent reaction time exceeds a second preset time, determining the reaction result in the standard control chamber. Wherein, in the standard control chamber, if the difference between the processing value of the brightness control signal corresponding to a blue light and the processing value of the brightness control signal corresponding to a green light is greater than a third preset difference, or the processing value of the brightness control signal corresponding to a red light and the processing value of the brightness control signal corresponding to a green light is greater than a fourth preset difference, determining that the reaction result in the reagent reaction chamber is positive; if the reagent reaction time exceeds a third preset time, and the difference between the processing value of the brightness control signal corresponding to a blue light and the processing value of the brightness control signal corresponding to a green light is less than or equal to the third preset difference, or if the processing value of the brightness control signal corresponding to a red light and the processing value of the brightness control signal corresponding to a green light is less than or equal to the fourth preset difference, determining that the reaction result in the reagent reaction chamber is invalid.

2. when the difference between the processing value of the brightness control signal corresponding to a blue light and the processing value of the brightness control signal corresponding to a green light is less than or equal to the first preset difference, or the difference between the processing value of the brightness control signal corresponding to a red light and the processing value of the brightness control signal corresponding to a green light is less than or equal to the second preset difference, if the reagent reaction time exceeds the second preset time, determining the reaction result in the standard control chamber. Wherein, in the standard control chamber, if the difference between the processing value of the brightness control signal corresponding to a blue light and the processing value of the brightness control signal corresponding to a green light is greater than the third preset difference, or the difference between the processing value of the brightness control signal corresponding to a red light and the processing value of the brightness control signal corresponding to a green light is greater than the fourth preset difference, determining that the reaction result in the reagent reaction chamber is negative; if the reagent reaction time exceeds the third preset time, and the difference between the processing value of the brightness control signal corresponding to a blue light and the processing value of the brightness control signal corresponding to a green light is less than or equal to the third preset difference, or if the difference between the processing value of the brightness control signal corresponding to a red light and the processing value of the brightness control signal corresponding to a green light is less than or equal to the fourth preset difference, determining that the reaction result in the reagent reaction chamber is invalid.

In some embodiments of the present disclosure, the method further comprises: after the reagent reaction time exceeds the first preset time, recording the brightness control signal corresponding to a blue light and the brightness control signal corresponding to a green light at every moment; calculating the rate of change of the brightness control signal corresponding to a blue light at adjoining times, and/or the rate of change of the brightness control signal corresponding to a green light at adjoining times; when an absolute value of the rate of change is greater than a rate of change threshold, determining the reaction result in the reagent reaction chamber is invalid.

For the above purposes, in a second aspect of the present disclosure, some embodiments provide a computer-readable storage medium, wherein a reagent reaction determination program applied to a test tube is stored thereon, when the reagent reaction determination program applied to a test tube is executed by a processor, the reagent reaction determination method applied to a test tube according to the above embodiments is implemented.

A computer-readable storage medium in some embodiments of the present disclosure is capable of improving the accuracy of the reagent reaction determination, while simplifying the determination steps and reducing the determination time, which greatly improves the user experience by executing the reagent reaction determination program applied to a test tube is stored thereon.

For the above purposes, in a third aspect of the present disclosure, some embodiments provide a test tube, which comprises a memory, a processor, and a reagent reaction determination program applied to a test tube, the reagent reaction determination program being stored on the memory and operable on the processor, and when the processor executes the reagent reaction determination program applied to a test tube, the reagent reaction determination method applied to a test tube according to the above embodiments is implemented.

A test tube in some embodiments of the present disclosure comprises a memory and processor, while the processor executes the reagent reaction determination program applied to a test tube stored on the memory. It is capable of improving the accuracy of a reagent reaction determination, while simplifying the determination steps and reducing the determination time, which greatly improves the user experience.

For the above purposes, in a fourth aspect of the present disclosure, some embodiments provide a reagent reaction determination device applied to a test tube, wherein the test tube is internally provided with a sampling chamber suitable for accommodating a sampling tube, at least one reagent reaction chamber is formed in the bottom panel of the sampling chamber. And each of the reagent reaction chamber is correspondingly provided with a light-incident component and a light-emergent component, the light-incident component guides RGB light to the reagent reaction chamber, and the light-emergent component guides a light passing through the reagent reaction chamber. The device comprises: a brightness control module, configured to output a brightness control signal to the light-incident component to control the light-incident component to emit the RGB light, and to adjust the brightness control signal according to a preset step length to adjust the brightness of the RGB light; a detection module, configured to detect brightness variations of the light derived by the light-emergent component to generate an electrical signal detection value; a determination module, configured to perform an interpolation calculation on the present brightness control signal to obtain an estimated value of the brightness control signal when the electrical signal detection value is less than a preset threshold, and to determine a reaction result in the reagent reaction chamber according to the estimated value of the brightness control signal.

In a reagent reaction determination device applied to a test tube in some embodiments of the present disclosure, the test tube is internally provided with a sampling chamber suitable for accommodating a sampling tube, at least one reagent reaction chamber is formed in the bottom panel of the sampling chamber. And each of the reagent reaction chamber is correspondingly provided with a light-incident component and a light-emergent component, the light-incident component guides RGB light to the reagent reaction chamber, and the light-emergent component guides the RGB light passing through the reagent reaction chamber. A reagent reaction determination device applied to a test tube in some embodiments of the present disclosure comprises a brightness control module, a detection module and a determination module, wherein, firstly, the brightness control module is configured to output a brightness control signal to the light-incident component to control the light-incident component to emit the RGB light, and to adjust the brightness control signal according to a preset step length, so as to adjust the brightness of the RGB light; then the detection module is configured to detect variations in the brightness of the light derived by the light-emergent component to generate an electrical signal detection value; the determination module is configured to perform an interpolation calculation on the present brightness control signal to obtain an estimated value of the brightness control signal when the electrical signal detection value is less than a preset threshold, and to determine a reaction result in the reagent reaction chamber according to the estimated value of the brightness control signal. Therefore, the reagent reaction determination device applied to a test tube in some embodiments of the present disclosure is capable of improving the accuracy of a reagent reaction determination, while simplifying the determination steps and reducing the determination time, which greatly improves the user experience.

Additional aspects and advantages of the present disclosure will be given in part in the following description, portions of which will become apparent from the following description or will be learned through the practice of the present disclosure.

## Brief Description of the Drawings

The above and/or additional aspects and advantages of the present disclosure will become apparent and easily understood from the description of the embodiments with the following drawings, wherein:

Fig. 1 shows a structural diagram of a sampling tube and a test tube in one embodiment of the present disclosure;

Fig. 2 shows a package structural diagram of a sampling tube and a test tube in one embodiment of the present disclosure;

Fig. 3 shows a structural diagram of a test tube in one embodiment of the present disclosure;

Fig. 4 shows a flow chart of a reagent reaction determination method in one embodiment of the present disclosure;

Fig. 5-1 shows a RGB color value diagram with negative reaction result in one embodiment of the present disclosure;

Fig. 5-2 shows a RGB color value diagram with positive reaction result in one embodiment of the present disclosure;

Fig. 6 shows a flow chart of a reagent reaction determination method in one specific embodiment of the present disclosure;

Fig. 7 shows a flow chart of a reagent reaction determination method in another specific embodiment of the present disclosure;

Fig. 8 shows a structural block diagram of a test tube in one embodiment of the present disclosure;

Fig. 9 shows a structural block diagram of a reagent reaction determination device applied to a test tube in one embodiment of the present disclosure;

## Detailed Description of the Embodiments

Embodiments of the present disclosure are described in detail below, and examples of the embodiments are shown in the drawings, wherein the same or similar symbols from first to last represent the same or similar elements or elements with same or similar functions. It should be appreciated that the specific embodiments described herein are only intended to explain the present disclosure, rather than limiting the protection scope of the present disclosure.

The following describes a reagent reaction determination method and device applied to a test tube, a computer-readable storage medium and a test tube in some embodiments of the present disclosure.

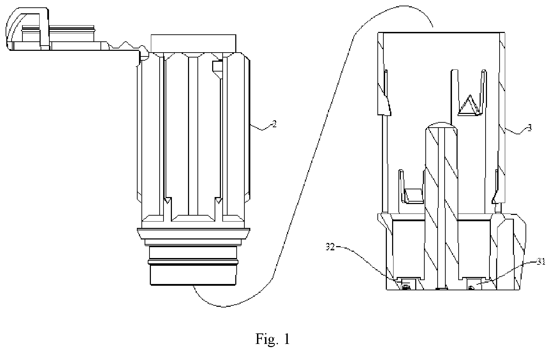

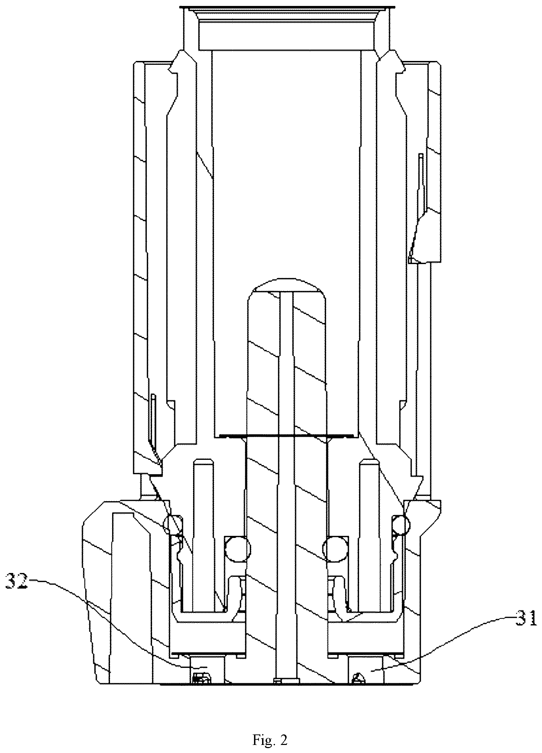

See Fig. 1 for an illustration. Fig. 1 shows a sampling tube 2 and a test tube 3, wherein a cotton swab may be placed in the sampling tube 2, and nucleic acids from a person being tested can be collected on the cotton swab, and the method of collecting the nucleic acids may include but not limited to taking throat swabs, collecting sputum, or lower respiratory tract specimens and others. After the nucleic acid sample has been collected, the cotton swab can be placed in the sampling tube 2 for LAMP (Loop-mediated isothermal amplification). The test tube 3 is internally provided with a sampling chamber suitable for accommodating the sampling tube 2, as shown in Fig. 2, and at least one reagent reaction chamber is formed in the bottom panel of the sampling chamber. Two reagent reaction chambers are identified in Fig. 1, namely a reaction chamber 31 and a reaction chamber 32.

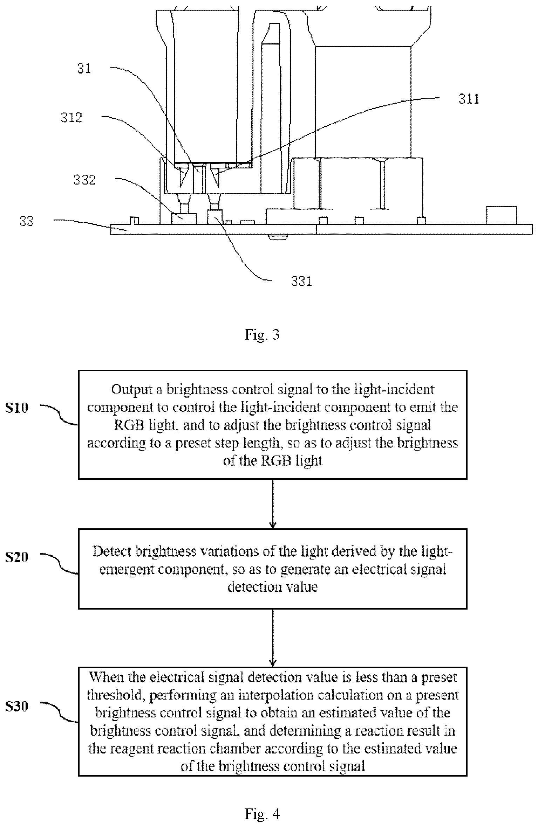

After placing the cotton swab into the sampling tube 2, the sampling tube 2 can be placed in the test tube 3, and the reagent can flow from the sampling tube 2 into each of the reagent reaction chamber, as shown in Fig. 3. Each of the reagent reaction chamber is correspondingly provided with a light-incident component 331 and a light-emergent component 332 (only the reagent reaction chamber 31 is shown herein). And, each of the reagent reaction chamber is correspondingly provided with a first prism 311 and a second prism 312. The light-incident component 331 emits RGB light, and the RGB light is guided to the reagent reaction chamber by the first prism 311, and the light-emergent component 332 guides a light passing through the reagent reaction chamber.

Specifically, the first prism 311 and the second prism 312 in the embodiment can be used to change the optical path of the RGB light so that the RGB light can irradiate the detection reagent in the reagent reaction chamber. Further, as shown in Fig. 3, wherein the test tube 3 also comprises a printed circuit board 33, the light-incident component 331 may be provided on the printed circuit board 33, and optionally, the light-incident component 331 may be an LED (light-emitting diode) lamp. See Fig. 3 for an illustration. The light-incident component 331 emits the RGB light which enters the reagent reaction chamber 31 after being reflected by the first prism 311 and is irradiated to the second prism 312, and then the second prism 312 reflects the RGB light to the light-emergent component 332 set on the printed circuit board 33. The light-emergent component 332 in the embodiment may be a photodiode, and in turn according to the brightness of the RGB light, an electrical signal may be converted by the light-emergent component 332.

Fig. 4 shows a flow chart of a reagent reaction determination method in one embodiment of the present disclosure;

As shown in Fig. 4, the reagent reaction determination method applied to a test tube in the embodiment comprises the following steps:

S10, output a brightness control signal to the light-incident component 331, so as to control the light-incident component 331 to emit the RGB light, and adjust the brightness control signal according to a preset step length, so as to adjust the brightness of the RGB light.

Specifically, the embodiment can control the brightness control signal of the light-incident component 331, which in turn enables the light-incident component 331 to emit a RGB light corresponding to the brightness control signal. And, the brightness control signal in the embodiment is adjusted according to a preset step length, such as adjusting the electromotion frequency of the light-incident component 331 by PWM (Pulse width modulation), the preset step length may be 10%, i.e., the PWM of the light-incident component 331 may be controlled to boost from 0 each time in step length of 10%. And in the embodiment, it may boost up to 100% and in turn can adjust the RGB light for different brightness.

S20, detect brightness variations of the light derived by the light-emergent component 332, so as to generate an electrical signal detection value.

Specifically, after adjusting the light-incident component 331 for different brightness, the light-emergent component 332 may generate different electrical signal detection values according to different light brightness. It will be appreciated that after the light-emergent component 331 receives the brightness control signal to change the brightness of the RGB light, the light-emergent component 332 may wait for a preset time (e.g., 1 second) before generating the corresponding electrical signal detection value based on the changed brightness of the RGB light, so as to ensure that the light received by the light-emergent component 332 is stable in terms of light brightness, which in turn may generate an accurate electrical signal detection value.

S30, when the electrical signal detection value is less than a preset threshold, perform an interpolation calculation on a present brightness control signal to obtain an estimated value of the brightness control signal, and determine a reaction result in the reagent reaction chamber according to the estimated value of the brightness control signal.

Specifically, after the light-emergent component 332 generates an electrical signal detection value, the electrical signal detection value may be determined, if the electrical signal detection value is determined to be less than a preset threshold, then perform an interpolation calculation on a present brightness control signal to obtain an estimated value of the brightness control signal, wherein, the preset threshold is optionally 1500 microamps. In the embodiment, if the electrical signal detection value is determined to be greater than or equal to the preset threshold, record the present electrical signal detection value and return to continue adjusting the brightness control signal, i.e., to increase the electromotion frequency of the light-incident component 331, i.e., the PWM value, until the electrical signal detection value is less than the preset threshold.

More specifically, the interpolation calculation performed on a brightness control signal in the embodiment may be a linear interpolation calculation. And, it should be noted that the electrical signal detection value in the present embodiment is inversely proportional to the brightness control signal, i.e., the larger the PWM value of the brightness control signal, the smaller the corresponding electrical signal detection value.

After performing an interpolation calculation on a present brightness control signal and obtaining an estimated value of the brightness control signal, a reaction result in the reagent reaction chamber may be determined according to the estimated value of the brightness control signal obtained by the interpolation calculation, and it can be determined whether the person to be detected tested is positive or negative.

In one embodiment of the present disclosure, in step S30 above, perform an interpolation calculation on a present brightness control signal, which may comprise: according to the preset step length, the preset threshold, and a present electrical signal detection value, a present value of the brightness control signal and a last electrical signal detection value when the electrical signal detection value is less than the preset threshold.

Specifically, the interpolation calculation performed on a brightness control signal in the embodiment may be a linear interpolation calculation, so the slopes are identical, thus the value of the brightness control signal corresponding to the preset threshold can be derived based on the slope and the preset threshold value. Wherein, the slopes can be calculated based on the preset step length, a present electrical signal detection value, a present value of the brightness control signal and a last electrical signal detection value when the electrical signal detection value is less than the preset threshold. After the corresponding slope is calculated, the estimated value of the brightness control signal can be determined based on the preset threshold.

More specifically, in the embodiment, the estimated value of the brightness control signal is calculated according to the following formula: $Y=Y1+a*[(X0-X1)/(X-X1)]$, wherein $Y$ is the estimated value of the brightness control signal, $Y1$ is the present value of the brightness control signal, $a$ is the preset step length, $X0$ is the preset threshold, $X1$ is the last electrical signal detection value, $X$ is the present electrical signal detection value.

For example, in the embodiment, the preset threshold $X0$ is 1500 microamps, the preset step length $a$ is 10, the present electrical signal detection value $X$ is 1498, the corresponding present value of the brightness control signal $Y1$ is 90, the last electrical signal detection value $X1$ is 1502, according to the above formula, the estimated value of the brightness control signal is calculated as $Y=90+10*[(1500-1502)/(1498-1502)]=95$. In the embodiment, after the estimated value of the brightness control signal is calculated, the corresponding reaction result in the reagent reaction chamber can be determined as positive or negative based on the estimated value of the brightness control signal. It is to be noted that a comparison value may be set to be compared with the estimated value of the brightness control signal, so that a reaction result in the reagent reaction chamber may be obtained according to the comparison result.

In some embodiments of the present disclosure, in step S30 above, when obtaining the estimated value of the brightness control signal, the method further comprises: determining a reagent reaction time, and when the reagent reaction time exceeds a first preset time, performing a standardized processing on the estimated value of the brightness control signal to obtain a standardized processing value, so as to determine the reaction result in the reagent reaction chamber according to the standardized processing value.

Specifically, as it was found during the experiments that the electrical signal detection value was very unstable before the first preset time, due to the presence of interference such as reagent reaction temperature and RGB light brightness error. Therefore, it is easy to lead to the situation that the electrical signal detection value before the first preset time is unstable. Therefore, in this embodiment, the reagent reaction time is also determined, and only when the reagent reaction time exceeds the first preset time can it be applied to the subsequent steps. Optionally, when the reagent reaction time does not exceed the first preset time, return to the step S10, and boost the brightness control signal in accordance with the preset step length. It is noted that the first preset time in this embodiment may be obtained by a statistical experiment, and optionally, the first preset time may be 15 minutes. It is further noted that the first preset time in this embodiment is counted from the time when the cotton swab is placed in the sampling tube, i.e., when performing LAMP with the nucleic acid sample, the first preset time in this embodiment is counted.

Further, because the reaction before the first preset time does not have reference value, this embodiment also comprises: when the reagent reaction time exceeds the first preset time, performing a standardized processing on the estimated value of the brightness control signal to obtain a standardized processing value, and determining the reaction result in the actual reagent reaction chamber according to the standardized processing value. It will be appreciated that the influence of the unstable signal before the first preset time on the determination of the reaction result can be reduced after the standardized processing, and the accuracy of the determination of the reaction result can be improved.

In this embodiment, performing the standardized processing on the estimated value of the brightness control signal comprises: determining the sum of the estimated value of the present brightness control signal and the estimated value of the brightness control signal within an effective reaction time, calculating an average value of the brightness control signal within the effective reaction time, and dividing the estimated value of the present brightness control signal by the average value of the brightness control signal within the effective reaction time, so as to obtain the standardized processing value.

After the reagent has reacted for more than 15 minutes, perform the standardized processing on the estimated value of the present brightness control signal. Specifically, the reagent reaction before a tenth minute can be ignored and the period from the tenth minute to the fifteenth minute can be taken as an effective reaction time, then calculate an average value of the brightness control signal within the effective reaction time. Specifically, the average value of the brightness control signal per second or the average value of the brightness control signal per minute can be derived according to the actual application. The present estimated value of the brightness control signal is divided by the average value of the brightness control signal obtained above to obtain a corresponding percentage value, which is the standardized processing value corresponding to the estimated value of the brightness control signal after the standardized processing.

Both the estimated value of the brightness control signal and the standardized processing value are recorded at all times, so that in the effective reaction time, the estimated value of the brightness control signal at every moment is recorded, and an average value of the brightness control signal during the period of time is derived, and then the estimated value of the brightness control signal subsequently recorded can be divided by that average value to obtain the standardized processing value.

In some embodiments of the present disclosure, the reagent reaction determination method further comprises: after the reagent reaction time exceeds the first preset time, recording the brightness control signal corresponding to a blue light and the brightness control signal corresponding to a green light at every moment; calculating the rate of change of the brightness control signal corresponding to a blue light at adjoining times, and/or the rate of change of the brightness control signal corresponding to a green light at adjoining times; when an absolute value of the rate of change is greater than a rate of change threshold, determining that the reaction result in the reagent reaction chamber is invalid.

Specifically, after determining that the reagent reaction time exceeds the first preset time, such as exceeding 15 minutes, record the brightness control signal corresponding to a blue light and the brightness control signal corresponding to a green light at every moment. More specifically, the brightness control signal corresponding to the blue light at 15 minutes 01 seconds, 15 minutes 02 seconds, etc., may be recorded, and then the rate of change of the brightness control signal corresponding to a blue light at adjoining time may be calculated. For example, the brightness control signal corresponding to the blue light at 15 minutes 01 seconds is 52, and the brightness control signal corresponding to the blue light at 15 minutes 02 seconds is 58, then the rate of change of the brightness control signal corresponding to a blue light at adjoining time is calculated as $| (58-52)/52 |= 0.12$. Optionally, a rate of change threshold in this embodiment is 0.25, when the absolute value of the rate of change of the brightness control signal corresponding to a blue light at adjoining time is greater than 0.25. Then it can be determined that the test tube has a mechanical failure or noise interference caused by reflux, etc., during the testing process, and thus the reaction result can be determined to be invalid. It will be appreciated that the determination method of the rate of change of the brightness control signal corresponding to a green light at adjoining times can be seen in the determination method of the rate of change of the brightness control signal corresponding to a blue light at adjoining times in the above-described embodiment, and will not be repeated herein.

In some embodiments of the present disclosure, the standardized processing value comprises a processing value of the brightness control signal corresponding to a blue light and a processing value of the brightness control signal corresponding to a green light in the RGB light, or a processing value of the brightness control signal corresponding to a red light and a processing value of the brightness control signal corresponding to a green light in the RGB light. Wherein, determining the reaction result in the reagent reaction chamber according to the standardized processing value comprises: when the difference between the processing value of the brightness control signal corresponding to a blue light and the processing value of the brightness control signal corresponding to a green light is greater than a first preset difference, or the processing value of the brightness control signal corresponding to a red light and the processing value of the brightness control signal corresponding to a green light is greater than a second preset difference, determining that the reaction result in the reagent reaction chamber is positive.

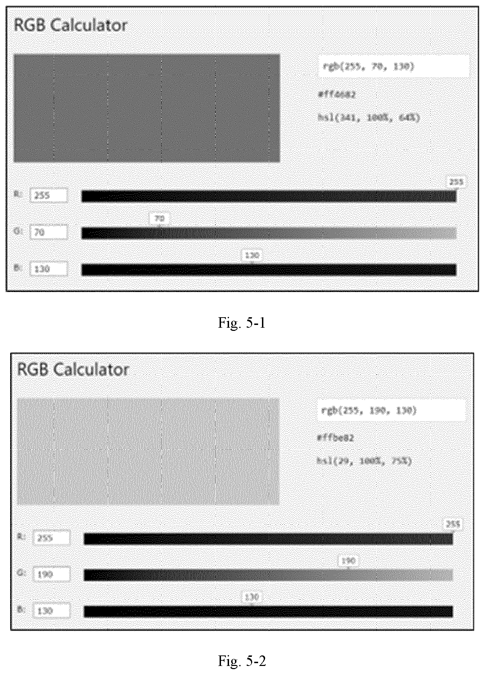

As shown in Fig. 5-1 and Fig. 5-2, among the reagent RGB values with negative detecting results and the reagent RGB values with positive detecting results, it is the color value corresponding to green light that changes significantly, while the other red light and blue light remain basically unchanged. Thus, in this embodiment, a positive or negative reaction result in the reagent reaction chamber can be determined by determining the difference in color values between the blue light and the green light, and/or the difference in color values between the red light and the green light.

Specifically, the standardized processing value comprises a processing value of the brightness control signal corresponding to a blue light and a processing value of the brightness control signal corresponding to a green light in the RGB light, or a processing value of the brightness control signal corresponding to a red light and a processing value of the brightness control signal corresponding to a green light in the RGB light. Further, determining the reaction result in the reagent reaction chamber according to the standardized processing value comprises: when the difference between the processing value of the brightness control signal corresponding to a blue light and the processing value of the brightness control signal corresponding to a green light is greater than a first preset difference, or the processing value of the brightness control signal corresponding to a red light and the processing value of the brightness control signal corresponding to a green light is greater than a second preset difference, determining that the reaction result in the reagent reaction chamber is positive. It is noted that the first preset difference and the second preset difference in the embodiment are different, and the first preset difference and the second preset difference are obtained through a number of statistical experiments, and optionally, the first preset difference may be any one of 105-107.

It is noted that in the embodiment, if the difference between the processing value of the brightness control signal corresponding to a blue light and the processing value of the brightness control signal corresponding to a green light is less than the first preset difference, determine that the reaction result in the reagent reaction chamber is negative; or, if the processing value of the brightness control signal corresponding to a red light and the processing value of the brightness control signal corresponding to a green light is less than the second preset difference, determine that the reaction result in the reagent reaction chamber is negative.

In the embodiment, there are a plurality of reagent reaction chambers, and one of the reagent reaction chambers is a standard control chamber, and determining that the reaction result in the reagent reaction chamber comprises:

1.when the difference between the processing value of the brightness control signal corresponding to a blue light and the processing value of the brightness control signal corresponding to a green light is greater than the first preset difference, or the processing value of the brightness control signal corresponding to a red light and the processing value of the brightness control signal corresponding to a green light is greater than the second preset difference, if the reagent reaction time exceeds a second preset time, determining the reaction result in the standard control chamber. Wherein, in the standard control chamber, if the difference between the processing value of the brightness control signal corresponding to a blue light and the processing value of the brightness control signal corresponding to a green light is greater than a third preset difference, or the processing value of the brightness control signal corresponding to a red light and the processing value of the brightness control signal corresponding to a green light is greater than a fourth preset difference, determining that the reaction result in the reagent reaction chamber is positive. If the reagent reaction time exceeds a third preset time, and the difference between the processing value of the brightness control signal corresponding to a blue light and the processing value of the brightness control signal corresponding to a green light is less than or equal to the third preset difference, or if the processing value of the brightness control signal corresponding to a red light and the processing value of the brightness control signal corresponding to a green light is less than or equal to the fourth preset difference, determining that the reaction result in the reagent reaction chamber is invalid.

2.when the difference between the processing value of the brightness control signal corresponding to a blue light and the processing value of the brightness control signal corresponding to a green light is less than or equal to the first preset difference, or the difference between the processing value of the brightness control signal corresponding to a red light and the processing value of the brightness control signal corresponding to a green light is less than or equal to the second preset difference, if the reagent reaction time exceeds the second preset time, determining the reaction result in the standard control chamber. Wherein, in the standard control chamber, if the difference between the processing value of the brightness control signal corresponding to a blue light and the processing value of the brightness control signal corresponding to a green light is greater than the third preset difference, or the difference between the processing value of the brightness control signal corresponding to a red light and the processing value of the brightness control signal corresponding to a green light is greater than the fourth preset difference, determining that the reaction result in the reagent reaction chamber is negative. If the reagent reaction time exceeds the third preset time, and the difference between the processing value of the brightness control signal corresponding to a blue light and the processing value of the brightness control signal corresponding to a green light is less than or equal to the third preset difference, or if the difference between the processing value of the brightness control signal corresponding to a red light and the processing value of the brightness control signal corresponding to a green light is less than or equal to the fourth preset difference, determining that the reaction result in the reagent reaction chamber is invalid.

Specifically, in this embodiment, the primer in the standard control chamber are different from that in the other reagent reaction chambers, but the determination of the reaction result is the same. The difference is that in the standard control chamber, if the reagent reaction result is positive, it means that the present reagent reaction is valid. The nucleic acid detecting result of the person to be detected can be determined based on the reaction result in the reagent reaction chamber, but if the reagent reaction result in the standard control chamber is negative, it means that the present reagent reaction result is invalid. And it is necessary to recollect the nucleic acid of the corresponding person to be detected, and repeat the above steps for the detection. The specific determination results are shown in Table 1:

Table 1

|  | standard control chamber positive | standard control chamber negative |
|---|---|---|
| reagent reaction chamber positive | positive | invalid |
| reagent reaction chamber negative | negative | invalid |

More specifically, in plurality of the reagent reaction chambers, when the difference between the processing value of the brightness control signal corresponding to a blue light and the processing value of the brightness control signal corresponding to a green light is less than or equal to the first preset difference, or the difference between the processing value of the brightness control signal corresponding to a red light and the processing value of the brightness control signal corresponding to a green light is less than or equal to the second preset difference. At this point it is not possible to determine that the corresponding detecting result for the person to be detected is negative. It is necessary to further determine whether the reagent reaction time exceeds the second preset time. If the reagent reaction time exceeds the second preset time, the corresponding detecting result of the person to be detected can be determined based on the reaction result in the standard control chamber. Wherein, the second preset time may be obtained through a number of experiments, optionally, the second preset time may be considered to be the time when the complete reaction of the sample in the reagent reaction chamber is finished. Optionally, the second preset time may be 30 minutes.

More specifically, in the process of determining reagent reaction time, if the reagent reaction time exceeds the second preset time, determine the reaction result in the standard control chamber. Wherein, in the standard control chamber, if the difference between the processing value of the brightness control signal corresponding to a blue light and the processing value of the brightness control signal corresponding to a green light is greater than the third preset difference, or the difference between the processing value of the brightness control signal corresponding to a red light and the processing value of the brightness control signal corresponding to a green light is greater than the fourth preset difference, then it means that the actual reaction result in the present standard control chamber is positive, and the present detecting result is valid. Therefore, the final detecting result can be determined based on the detecting result in the reagent reaction chamber, i.e., the final detecting result is negative. Optionally, the third and fourth preset differences are any one of 101-107. Preferably, the third preset difference is 101.

If the reagent reaction time exceeds the third preset time, and the difference between the processing value of the brightness control signal corresponding to a blue light and the processing value of the brightness control signal corresponding to a green light is less than or equal to the third preset difference, or if the processing value of the brightness control signal corresponding to a red light and the processing value of the brightness control signal corresponding to a green light is less than or equal to the fourth preset difference, then it means that the actual reaction result in the present standard control chamber is negative, and the present detecting result is invalid. Wherein, the third preset time may be considered to be the time when the complete reaction of the sample in the reagent reaction chamber is finished. Optionally, the third preset time may be 35 minutes.

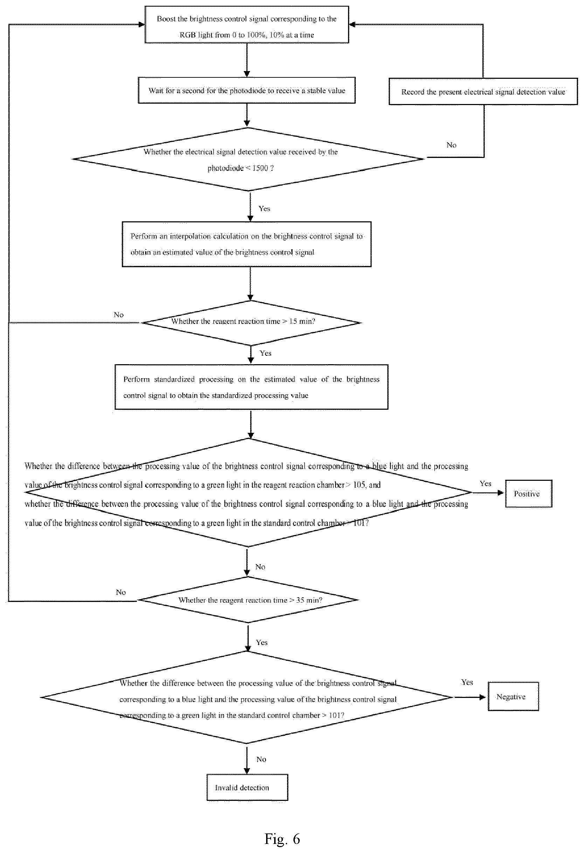

Fig. 6 shows a flow chart of a reagent reaction determination method in one specific embodiment of the present disclosure.

Specifically, firstly, boost the brightness control signal corresponding to the RGB light from 0, by 10% each time, wait for 1 second so that the photodiode is able to receive a stable electrical signal detection value, and then determine whether the electrical signal detection value received by the photodiode is less than 1500 microamps. If so, perform a linear interpolation calculation on the brightness control signal to obtain an estimated value of the brightness control signal; if not, record the present electrical signal detection value and return to the first step to boost the brightness control signal again. After obtaining the estimated value of the brightness control signal, determine whether the reagent reaction time is greater than 15 minutes. If so, perform the standardized processing on the estimated value of the brightness control signal to obtain the standardized processing value; if not, record the present electrical signal detection value and return to the first step to boost the brightness control signal again. After obtaining the standardized processing value, determine whether the difference between the processing value of the brightness control signal corresponding to a blue light and the processing value of the brightness control signal corresponding to a green light in the reagent reaction chamber is greater than 105, and whether the difference between the processing value of the brightness control signal corresponding to a blue light and the processing value of the brightness control signal corresponding to a green light in the standard control chamber is greater than 101. If both are greater, a positive detecting result is obtained; if neither are greater, then further determine whether the reagent reaction time is greater than 35 minutes. If the reagent reaction time is not greater than 35 minutes, then return to the first step to boost the brightness control signal again; if greater than 35 minutes, then determine whether the difference between the processing value of the brightness control signal corresponding to a blue light and the processing value of the brightness control signal corresponding to a green light in the standard control chamber is greater than 101. If so, a negative detecting result is obtained; if not, the detection is invalid.

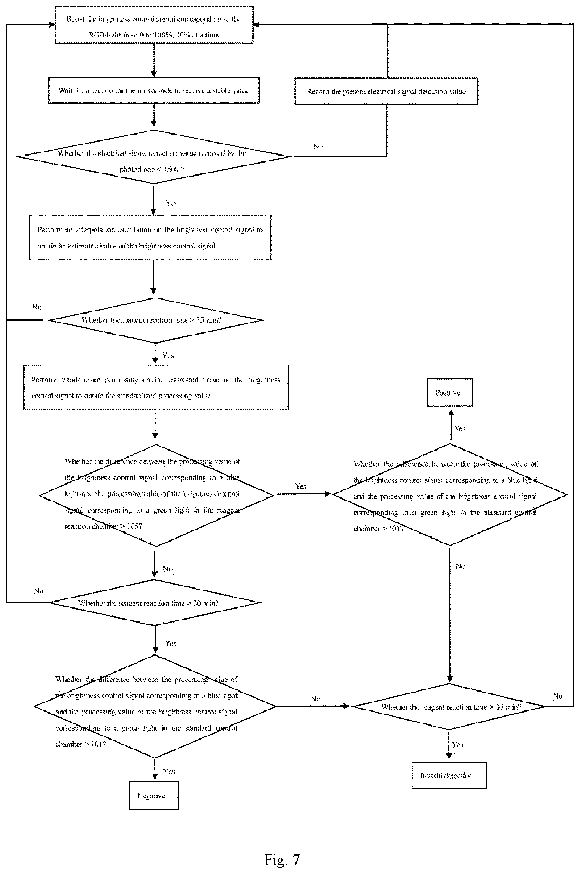

Fig. 7 shows a flow chart of a reagent reaction determination method in another specific embodiment of the present disclosure.

As shown in Fig. 7, firstly, boost the brightness control signal corresponding to the RGB light from 0, by 10% each time, wait for 1 second so that the photodiode is able to receive a stable electrical signal detection value, and then determine whether the electrical signal detection value received by the photodiode is less than 1500 microamps. If so, perform a linear interpolation calculation on the brightness control signal to obtain an estimated value of the brightness control signal; if not, record the present electrical signal detection value and return to the first step to boost the brightness control signal again. After obtaining the estimated value of the brightness control signal, determine whether the reagent reaction time is greater than 15 minutes. If so, perform the standardized processing on the estimated value of the brightness control signal to obtain the standardized processing value; if not, record the present electrical signal detection value and return to the first step to boost the brightness control signal again. After obtaining the standardized processing value, determine whether the difference between the processing value of the brightness control signal corresponding to a blue light and the processing value of the brightness control signal corresponding to a green light in the reagent reaction chamber is greater than 105. And if so, further determine whether the difference between the processing value of the brightness control signal corresponding to a blue light and the processing value of the brightness control signal corresponding to a green light in the standard control chamber is greater than 101. If so, a positive detecting result is obtained; if not, then further determine whether the reagent reaction time is greater than 35 minutes. If the reagent reaction time is not greater than 35 minutes, then return to the first step to boost the brightness control signal again; if greater than 35 minutes, then the present detection is invalid.

After obtaining the standardized processing value, if it is determined that the difference between the processing value of the brightness control signal corresponding to the blue light and the green light in the reagent reaction chamber is not greater than 105, then determine whether the reagent reaction time is greater than 30 minutes. If the reagent reaction time is not greater than 30 minutes, return to the first step to boost the brightness control signal again. If the reagent reaction time is greater than 30 minutes, then further determine whether the difference between the processing value of the brightness control signal corresponding to the blue light and the green light in the standard control chamber is greater than 101. If it is also greater than 101, then the detecting result is negative. If the difference between the processing value of the brightness control signal corresponding to the blue light and the green light in the standard control chamber is not greater than 101, then further determine whether the reagent reaction time is greater than 35 minutes. If it is not greater than 35 minutes, then return to the first step to boost the brightness control signal again; if it is greater than 35 minutes, the present detection is invalid.

Therefore, the reagent reaction determination method applied to a test tube in the embodiments of the present disclosure is capable of improving the accuracy of a reagent reaction determination, while simplifying the determination steps and reducing the determination time, which greatly improves the user experience.

Further, the present disclosure presents a computer-readable storage medium, wherein a reagent reaction determination program applied to a test tube is stored thereon, when the reagent reaction determination program applied to a test tube is executed by a processor, the reagent reaction determination method applied to a test tube according to the above embodiments is implemented.

A computer-readable storage medium in some embodiments of the present disclosure is capable of improving the accuracy of a reagent reaction determination, while simplifying the determination steps and reducing the determination time, which greatly improves the user experience by executing the reagent reaction determination program applied to a test tube stored thereon.

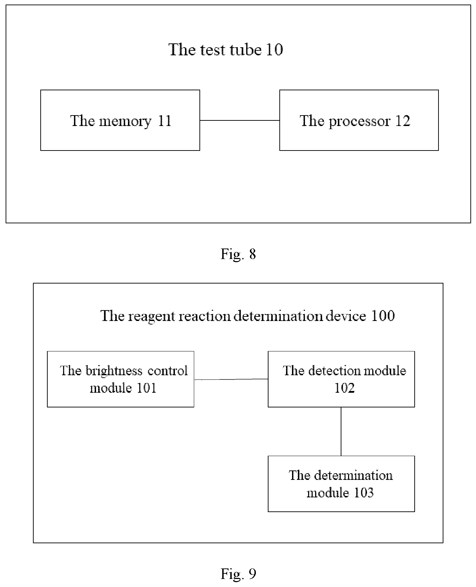

Fig. 8 shows a structural block diagram of a test tube in one embodiment of the present disclosure.

Further, as shown in Fig. 8, the present disclosure presents a test tube 10, which comprises a memory 11, a processor 12, and a reagent reaction determination program applied to a test tube, the reagent reaction determination program being stored on the memory 11 and operable on the processor 12, and when the processor 12 executes the reagent reaction determination program applied to a test tube, the reagent reaction determination method applied to a test tube according to the above embodiments is implemented.

A test tube in some embodiments of the present disclosure comprises a memory and a processor, and the processor executes the reagent reaction determination program applied to a test tube stored on the memory. It is capable of improving the accuracy of a reagent reaction determination, while simplifying the determination steps and reducing the determination time, which greatly improves the user experience.

Fig. 9 shows a structural block diagram of a reagent reaction determination device applied to a test tube in one embodiment of the present disclosure.

Further, as shown in Fig. 9, the present disclosure presents a reagent reaction determination device applied to a test tube. Wherein, as shown in Fig. 1-3, the test tube 3 is internally provided with a sampling chamber suitable for accommodating a sampling tube 2. At least one reagent reaction chamber is formed in the bottom panel of the sampling chamber, and each of the reagent reaction chamber is correspondingly provided with a light-incident component 331 and a light-emergent component 332. The light-incident component 331 guides a RGB light to the reagent reaction chamber, and the light-emergent component 332 guides a light passing through the reagent reaction chamber.

Specifically, see Fig. 1 for an illustration. Fig. 1 shows a sampling tube 2 and a test tube 3. A cotton swab may be placed in the sampling tube 2, and nucleic acids from the person being tested can be collected on the cotton swab. And the method of collecting the nucleic acids may include but not limited to taking throat swabs, collecting sputum, or lower respiratory tract specimens and others. After the nucleic acid sample has been collected, the cotton swab can be placed in the sampling tube 2 for LAMP (Loop-mediated isothermal amplification). The test tube 3 is internally provided with a sampling chamber suitable for accommodating the sampling tube 2, and at least one reagent reaction chamber is formed in the bottom panel of the sampling chamber. Two reagent reaction chambers are identified in the embodiment of Fig. 1, namely a reaction chamber 31 and a reaction chamber 32.

After placing the cotton swab into the sampling tube 2, the sampling tube 2 can be placed in the test tube 3, and the reagent can flow from the sampling tube 2 into each of the reagent reaction chamber. As shown in Fig. 3, each of the reagent reaction chamber is correspondingly provided with a light-incident component 331 and a light-emergent component 332 (only the reagent reaction chamber 31 is shown herein). And in the embodiment each of the reagent reaction chamber is correspondingly provided with a first prism 311 and a second prism 312. The light-incident component 331 emits RGB light and guides the RGB light to the reagent reaction chamber through the first prism 311, and the light-emergent component 332 guides a light passing through the reagent reaction chamber.

Specifically, the first prism 311 and the second prism 312 in the embodiment can be used to change the optical path of the RGB light so that the RGB light can irradiate the detection reagent in the reagent reaction chamber. Further, as shown in Fig. 3, wherein the test tube 3 also comprises a printed circuit board 33, the light-incident component 331 may be provided on the printed circuit board 33. Optionally, the light-incident component 331 may be an LED (light-emitting diode) lamp. See Fig. 3 for an illustration. The light-incident component 331 emits the RGB light which enters the reagent reaction chamber 31 after being reflected by the first prism 311 and is irradiated to the second prism 312, and then the second prism 312 reflects the RGB light to the light-emergent component 332 set on the printed circuit board 33. The light-emergent component 332 in the embodiment may be a photodiode, and in turn the light-emergent component 332 may be converted to an electrical signal based on the brightness of the RGB light.

See Fig. 9 for an illustration. A reagent reaction determination device 100 applied to a test tube in the embodiment comprises a brightness control module 101, a detection module 102 and a determination module 103.

Wherein, the brightness control module 101 is configured to output a brightness control signal to the light-incident component, so as to control the light-incident component to emit the RGB light, and to adjust the brightness control signal according to a preset step length to adjust the brightness of the RGB light. The detection module 102 is configured to detect brightness variations of the light derived by the light-emergent component to generate an electrical signal detection value. The determination module 103 is configured to perform an interpolation calculation on the present brightness control signal to obtain an estimated value of the brightness control signal when the electrical signal detection value is less than a preset threshold, and to determine a reaction result in the reagent reaction chamber according to the estimated value of the brightness control signal.

Specifically, the brightness control module 101 in the embodiment can control the brightness control signal of the light-incident component 331, which in turn enables the light-incident component 331 to emit a RGB light corresponding to the brightness control signal. And, the brightness control signal in the embodiment is adjusted according to a preset step length, such as adjusting the electromotion frequency of the light-incident component 331 by PWM (Pulse Width Modulation). The preset step length may be 10%, i.e., the PWM of the light-incident component 331 may be boost from 0, each time in step length of 10%. And in the embodiment, it may boost up to 100% and in turn can adjust the RGB light for different brightness.

After the brightness control module 101 adjusts the light-incident component 331 for different brightness, the detection module 102 may detect the different electrical signal detection values according to different light brightness generated by the light-emergent component 332. It will be appreciated that after the light-emergent component 331 receives the brightness control signal to change the brightness of the RGB light, the light-emergent component 332 may wait for a preset time (e.g., 1 second) before generating the corresponding electrical signal detection value based on the changed brightness of the RGB light, so as to ensure that the light received by the light-emergent component 332 is stable in terms of light brightness, which in turn may generate an accurate electrical signal detection value.

After the detection module 102 detects the electrical signal detection value generated by the light-emergent component 332, the electrical signal detection value may be determined by the determination module 103. If the electrical signal detection value is determined to be less than a preset threshold, then perform an interpolation calculation on a present brightness control signal to obtain an estimated value of the brightness control signal, wherein, the preset threshold is optionally 1500 microamps. In the embodiment, if the electrical signal detection value is determined to be greater than or equal to the preset threshold, record the present electrical signal detection value and return to continue adjusting the brightness control signal, i.e., to increase the electromotion frequency of the light-incident component 331, i.e., the PWM value, until the electrical signal detection value is less than the preset threshold.

More specifically, the interpolation calculation performed on a brightness control signal in the embodiment may be a linear interpolation calculation. And, it should be noted that the electrical signal detection value in the present embodiment is inversely proportional to the brightness control signal, i.e., the larger the PWM value of the brightness control signal, the smaller the corresponding electrical signal detection value.

After performing an interpolation calculation on a present brightness control signal and obtaining an estimated value of the brightness control signal, a reaction result in the reagent reaction chamber may be determined according to the estimated value of the brightness control signal obtained by the interpolation calculation. And it can be determined whether the person to be detected tested is positive or negative.

In an embodiment of the present disclosure, the determination module 103 is further configured to return to continue adjusting the brightness control signal until the electrical signal detection value is less than the preset threshold when the electrical signal detection value is greater than or equal to the preset threshold.

In an embodiment of the present disclosure, the determination module 103 is further configured to determine an estimated value of the brightness control signal according to the preset step length, the preset threshold, and a present electrical signal detection value, a present value of the brightness control signal and a last electrical signal detection value when the electrical signal detection value is less than the preset threshold.

In an embodiment of the present disclosure, the determination module 103 is further configured to calculate the estimated value of the brightness control signal according to the following formula: $Y=Y1+a*[(X0-X1)/(X-X1)]$, wherein Y is the estimated value of the brightness control signal, Y1 is the present value of the brightness control signal, a is the preset step length, X0 is the preset threshold, X1 is the last electrical signal detection value, X is the present electrical signal detection value.

In an embodiment of the present disclosure, the determination module 103 is further configured to determine a reagent reaction time. And when the reagent reaction time exceeds the first preset time, perform a standardized processing on the estimated value of the brightness control signal to obtain a standardized processing value, so as to determine the reaction result in the reagent reaction chamber according to the standardized processing value.

In an embodiment of the present disclosure, the determination module 103 is further configured to determine the sum of the estimated value of the present brightness control signal and the estimated value of the brightness control signal within an effective reaction time, calculate an average value of the brightness control signal within the effective reaction time, and divide the estimated value of the present brightness control signal by the average value of the brightness control signal within the effective reaction time, so as to obtain the standardized processing value.

In an embodiment of the present disclosure, the standardized processing value comprises a processing value of the brightness control signal corresponding to a blue light and a processing value of the brightness control signal corresponding to a green light in the RGB light, or a processing value of the brightness control signal corresponding to a red light and a processing value of the brightness control signal corresponding to a green light in the RGB light. Wherein, determining the reaction result in the reagent reaction chamber according to the standardized processing value, comprises: when the difference between the processing value of the brightness control signal corresponding to a blue light and the processing value of the brightness control signal corresponding to a green light is greater than a first preset difference, or the processing value of the brightness control signal corresponding to a red light and the processing value of the brightness control signal corresponding to a green light is greater than a second preset difference, determining that the reaction result in the reagent reaction chamber is positive.

It is noted that in the embodiment, if the difference between the processing value of the brightness control signal corresponding to a blue light and the processing value of the brightness control signal corresponding to a green light is less than the first preset difference, determine that the reaction result in the reagent reaction chamber is negative; or, if the processing value of the brightness control signal corresponding to a red light and the processing value of the brightness control signal corresponding to a green light is less than the second preset difference, determine that the reaction result in the reagent reaction chamber is negative.

In an embodiment of the present disclosure, when there are a plurality of reagent reaction chambers, and one of the reagent reaction chambers is a standard control chamber, the determination module 103 is further configured to:

1. when the difference between the processing value of the brightness control signal corresponding to a blue light and the processing value of the brightness control signal corresponding to a green light is greater than the first preset difference, or the processing value of the brightness control signal corresponding to a red light and the processing value of the brightness control signal corresponding to a green light is greater than the second preset difference, if the reagent reaction time exceeds the second preset time, determine the reaction result in the standard control chamber. Wherein, in the standard control chamber, if the difference between the processing value of the brightness control signal corresponding to a blue light and the processing value of the brightness control signal corresponding to a green light is greater than the third preset difference, or the processing value of the brightness control signal corresponding to a red light and the processing value of the brightness control signal corresponding to a green light is greater than the fourth preset difference, determine that the reaction result in the reagent reaction chamber is positive. If the reagent reaction time exceeds the third preset time, and the difference between the processing value of the brightness control signal corresponding to a blue light and the processing value of the brightness control signal corresponding to a green light is less than or equal to the third preset difference, or if the processing value of the brightness control signal corresponding to a red light and the processing value of the brightness control signal corresponding to a green light is less than or equal to the fourth preset difference, determine that the reaction result in the reagent reaction chamber is invalid.

2. when the difference between the processing value of the brightness control signal corresponding to a blue light and the processing value of the brightness control signal corresponding to a green light is less than or equal to the first preset difference, or the difference between the processing value of the brightness control signal corresponding to a red light and the processing value of the brightness control signal corresponding to a green light is less than or equal to the second preset difference, if the reagent reaction time exceeds the second preset time, determine the reaction result in the standard control chamber. Wherein, in the standard control chamber, if the difference between the processing value of the brightness control signal corresponding to a blue light and the processing value of the brightness control signal corresponding to a green light is greater than the third preset difference, or the difference between the processing value of the brightness control signal corresponding to a red light and the processing value of the brightness control signal corresponding to a green light is greater than the fourth preset difference, determine that the reaction result in the reagent reaction chamber is negative. If the reagent reaction time exceeds the third preset time, and the difference between the processing value of the brightness control signal corresponding to a blue light and the processing value of the brightness control signal corresponding to a green light is less than or equal to the third preset difference, or if the difference between the processing value of the brightness control signal corresponding to a red light and the processing value of the brightness control signal corresponding to a green light is less than or equal to the fourth preset difference, determine that the reaction result in the reagent reaction chamber is invalid.

In some embodiments of the present disclosure, the determination module 103 is further configured to, after the reagent reaction time exceeds the first preset time, record the brightness control signal corresponding to a blue light and the brightness control signal corresponding to a green light at every moment; calculating the rate of change of the brightness control signal corresponding to a blue light at adjoining times, and/or the rate of change of the brightness control signal corresponding to a green light at adj oining times. When an absolute value of the rate of change is greater than a rate of change threshold, determine the reaction result in the reagent reaction chamber is invalid.

It is noted that the specific implementation of the reagent reaction determination device applied to a test tube in the embodiments of the present disclosure can be found in the reagent reaction determination method applied to a test tube in the above embodiments, and will not be repeated herein.

Therefore, the reagent reaction determination device applied to a test tube in the embodiments of the present disclosure is capable of improving the accuracy of a reagent reaction determination, while simplifying the determination steps and reducing the determination time, which greatly improves the user experience.

It is noted that the logic and/or steps represented in the flow chart or otherwise described herein. For example, it may be considered to be a sequential list of executable instructions for implementing the logic functions, and may be specifically implemented in any computer-readable medium for use by, or combining with, an instruction-executing system, device, or apparatus (such as a computer-based system, a system including a processor, or other system that can take instructions from an instruction-executing system, device, or apparatus and execute them). In terms of this specification, a "computer-readable medium" may be any device that can contain, store, communicate, disseminate, or transmit a program for use by, or combining with, an instruction-executing system, device, or apparatus. More specific embodiments of computer-readable medium (a non-exhaustive list) include the following: an electrical connection with one or more wiring (electronic device), a portable computer disk cartridge (magnetic device), a random access memory (RAM), a read only memory (ROM), an erasable and programmable read-only memory (EPROM or flash memory), a fiber optic device and a compact disc read only memory (CDROM). In addition, the computer-readable medium may even be paper or other suitable medium on which said program may be printed, since the program can be obtained electronically, for example, by optical scanning of paper or other media, followed by editing, decoding or, if necessary, processing in other suitable ways, and then the program can be stored in the computer memory.

It will be appreciated that each portion of the present disclosure may be implemented with hardware, software, firmware, or their combinations. In the above embodiments, the plurality of steps or methods may be implemented with software or firmware stored in memory and executed by a suitable instruction-executing system. For example, if implemented with hardware, as in another embodiment, it may be implemented by any of the following techniques known in the art, or a combination thereof: discrete logic circuits with logic gate circuits for implementing logic functions on data signals, application specific integrated circuits with suitable combinational logic gate circuits, programmable gate array (PGA), field programmable gate array (FPGA), etc.

In the description of this specification, reference terms "an embodiment", "some embodiments", "examples", "specific examples", or "some examples" mean that the specific features, structures, materials, or characteristics described in conjunction with the embodiment or example are included in at least one embodiment or example of the present disclosure. In this specification, indicative representations of the above terms do not necessarily refer to the same embodiment or example. Moreover, the specific features, structures, materials, or characteristics described may be combined in any one or more of the embodiments or examples in a suitable manner.

In the description of the present disclosure, it is to be understood that the terms "center", "longitudinal", "lateral", "length ", "width", "thickness", "over", "under", "front", "back", "left", "right", "vertical ", "horizontal", "top", "bottom", "inside", "outside", "clockwise", "counterclockwise", "axial", " radial", "circumferential", and the like indicate orientations or positional relationships based on those shown in the figures. It is intended only to facilitate and simplify the description of the present disclosure, and does not indicate or imply that the device or element referred to must have a particular orientation, be constructed or operated in a particular orientation. Therefore, it cannot be construed as a limitation of the present disclosure.

Furthermore, the terms "first" and "second" are used for descriptive purposes only and are not to be understood as indicating or implying relative importance or implicitly indicating the number of technical features indicated. Thus, a feature defined with the terms "first" or "second" may expressly or implicitly include at least one such feature. In the description of the present disclosure, "plurality" means at least two, e.g., two, three, etc., unless expressly and specifically defined otherwise.

In the present disclosure, unless explicitly stated and defined otherwise, the terms "mounted", "connected", "connected", "fixed", and like terms are to be broadly understood. For example, it may be either fixedly connected, removably connected or integral; may be mechanically or electrically connected; may be a connection within two elements or an interaction between two elements unless explicitly defined otherwise. The specific meanings of the above terms in the present disclosure can be understood by those of ordinary skill in the art on a case-by-case basis.

In the present disclosure, unless explicitly stated and defined otherwise, a first feature being "over" or "under" a second feature may be that the first and second features are in direct contact, or that the first and second features are in indirect contact through an intermediary. Furthermore, the first feature being "above", "over" and "on top of" the second feature can mean that the first feature is directly above or diagonally above the second feature, or simply indicates that the horizontal height of the first feature level is higher than that of the second feature. The first feature being "under", "below" and "beneath" the second feature can mean that the first feature is directly below or diagonally below the second feature, or simply indicates that the horizontal height of the first feature level is lower than that of the second feature.

Although embodiments of the present disclosure have been shown and described above, it will be appreciated that the above embodiments are exemplary and are not to be construed as a limitation of the present disclosure. Those of ordinary skill in the art may make changes, modifications, substitutions and variations of the above embodiments within the scope of the present disclosure.

**Claims**

1. A reagent reaction determination method applied to a test tube, wherein the test tube is internally provided with a sampling chamber suitable for accommodating a sampling tube, at least one reagent reaction chamber is formed in the bottom panel of the sampling chamber. And each of the reagent reaction chamber is correspondingly provided with a light-incident component and a light-emergent component, the light-incident component guides RGB light to the reagent reaction chamber, and the light-emergent component guides a light passing through the reagent reaction chamber, the method comprises:

   outputting a brightness control signal to the light-incident component, so as to control the light-incident component to emit the RGB light, and adjusting the brightness control signal according to a preset step length, so as to adjust the brightness of the RGB light;
   detecting brightness variations of the light derived by the light-emergent component, so as to generate an electrical signal detection value;
   when the electrical signal detection value is less than a preset threshold, performing an interpolation calculation on a present brightness control signal to obtain an estimated value of the brightness control signal, and determining a reaction result in the reagent reaction chamber according to the estimated value of the brightness control signal.

2. The reagent reaction determination method according to claim 1, wherein when the electrical signal detection value is greater than or equal to the preset threshold, return to continue adjusting the brightness control signal until the electrical signal detection value is less than the preset threshold.

3. The reagent reaction determination method according to claim 2, wherein performing the interpolation calculation on the present brightness control signal comprises:
determining the estimated value of the brightness control signal according to the preset step length, the preset threshold, and a present electrical signal detection value, a present value of the brightness control signal and a last electrical signal detection value when the electrical signal detection value is less than the preset threshold.

4. The reagent reaction determination method according to claim 3, wherein the estimated value of the brightness control signal is calculated according to the following formula:

$$Y=Y1+a*[(X0-X1)/(X-X1)]$$

wherein Y is the estimated value of the brightness control signal, Y1 is the present value of the brightness control signal, a is the preset step length, X0 is the preset threshold, X1 is the last electrical signal detection value, X is the present electrical signal detection value.

5. The reagent reaction determination method according to any one of claims 1-4, wherein when obtaining the estimated value of the brightness control signal, the method further comprises:
determining a reagent reaction time, and when the reagent reaction time exceeds a first preset time, performing a standardized processing on the estimated value of the brightness control signal to obtain a standardized processing value, so as to determine the reaction result in the reagent reaction chamber according to the standardized processing value.

6. The reagent reaction determination method according to claim 5, wherein performing the standardized processing on the estimated value of the brightness control signal comprises:
determining the sum of the estimated value of the present brightness control signal and the estimated value of the brightness control signal within an effective reaction time, calculating an average value of the brightness control signal within the effective reaction time, and dividing the estimated value of the present brightness control signal by the average value of the brightness control signal within the effective reaction time, so as to obtain the standardized processing value.

7. The reagent reaction determination method according to claim 5, wherein the standardized processing value comprises a processing value of the brightness control signal corresponding to a blue light and a processing value of the brightness control signal corresponding to a green light in the RGB light, or a processing value of the brightness control signal corresponding to a red light and a processing value of the brightness control signal corresponding to a green light in the RGB light, wherein determining the reaction result in the reagent reaction chamber according to the standardized processing value, comprising:

when the difference between the processing value of the brightness control signal corresponding to a blue light and the processing value of the brightness control signal corresponding to a green light is greater than a first preset difference, or the processing value of the brightness control signal corresponding to a red light and the processing value of the brightness control signal corresponding to a green light is greater than a second preset difference, determining that the reaction result in the reagent reaction chamber is positive;
if the difference between the processing value of the brightness control signal corresponding to a blue light and the processing value of the brightness control signal corresponding to a green light is less than the first preset difference, determining that the reaction result in the reagent reaction chamber is negative; or, if the processing value of the brightness control signal corresponding to a red light and the processing value of the brightness control signal corresponding to a green light is less than the second preset difference, determining that the reaction result in the reagent reaction chamber is negative.

8. The reagent reaction determination method according to claim 7, wherein there are a plurality of reagent reaction chambers, and one of the reagent reaction chambers is a standard control chamber, and the method further comprises:

when the difference between the processing value of the brightness control signal corresponding to a blue light and the processing value of the brightness control signal corresponding to a green light is greater than the first preset difference, or the processing value of the brightness control signal corresponding to a red light and the processing value of the brightness control signal corresponding to a green light is greater than the second preset difference, if the reagent reaction time exceeds a second preset time, determining the reaction result in the standard control chamber, wherein in the standard control chamber,

if the difference between the processing value of the brightness control signal corresponding to a blue light and the processing value of the brightness control signal corresponding to a green light is greater than a third preset difference, or the processing value of the brightness control signal corresponding to a red light and the processing value of the brightness control signal corresponding to a green light is greater than a fourth preset difference, determining that the reaction result in the reagent reaction chamber is positive;

if the reagent reaction time exceeds a third preset time, and the difference between the processing value of the brightness control signal corresponding to a blue light and the processing value of the brightness control signal corresponding to a green light is less than or equal to the third preset difference, or if the processing value of the brightness control signal corresponding to a red light and the processing value of the brightness control signal corresponding to a green light is less than or equal to the fourth preset difference, determining that the reaction result in the reagent reaction chamber is invalid;

when the difference between the processing value of the brightness control signal corresponding to a blue light and the processing value of the brightness control signal corresponding to a green light is less than or equal to the first preset difference, or the difference between the processing value of the brightness control signal corresponding to a red light and the processing value of the brightness control signal corresponding to a green light is less than or equal to the second preset difference, if the reagent reaction time exceeds the second preset time, determining the reaction result in the standard control chamber, wherein in the standard control chamber,

if the difference between the processing value of the brightness control signal corresponding to a blue light and the processing value of the brightness control signal corresponding to a green light is greater than the third preset difference, or the difference between the processing value of the brightness control signal corresponding to a red light and the processing value of the brightness control signal corresponding to a green light is greater than the fourth preset difference, determining that the reaction result in the reagent reaction chamber is negative;

if the reagent reaction time exceeds the third preset time, and the difference between the processing value of the brightness control signal corresponding to a blue light and the processing value of the brightness control signal corresponding to a green light is less than or equal to the third preset difference, or if the difference between the processing value of the brightness control signal corresponding to a red light and the processing value of the brightness control signal corresponding to a green light is less than or equal to the fourth preset difference, determining that the reaction result in the reagent reaction chamber is invalid;

9. The reagent reaction determination method according to claim 5, wherein the method further comprises:

after the reagent reaction time exceeds the first preset time, record the brightness control signal corresponding to a blue light and the brightness control signal corresponding to a green light at every moment;
calculating the rate of change of the brightness control signal corresponding to a blue light at adjoining times, and/or the rate of change of the brightness control signal corresponding to a green light at adjoining times;
when an absolute value of the rate of change is greater than a rate of change threshold, determine that the reaction result in the reagent reaction chamber is invalid.

10. A computer-readable storage medium, wherein a reagent reaction determination program applied to a test tube is stored thereon,
when the reagent reaction determination program applied to a test tube is executed by a processor, the reagent reaction determination method applied to a test tube according to any one of claims 1-9 is implemented.

11. A test tube, wherein, comprises a memory, a processor, and a reagent reaction determination program applied to a test tube, the reagent reaction determination program being stored on the memory and operable on the processor, and when the processor executes the reagent reaction determination program applied to a test tube, the reagent reaction determination method applied to a test tube according to any one of claims 1-9 is implemented.

12. A reagent reaction determination device applied to a test tube, wherein the test tube is internally provided with a sampling chamber suitable for accommodating a sampling tube, at least one reagent reaction chamber is formed in the bottom panel of the sampling chamber. And each of the reagent reaction chamber is correspondingly provided with a light-incident component and a light-emergent component, the light-incident component guides RGB light to the reagent reaction chamber, and the light-emergent component guides a light passing through the reagent reaction chamber, the device comprises:

a brightness control module, configured to output a brightness control signal to the light-incident component to control the light-incident component to emit the RGB light, and to adjust the brightness control signal according to a preset step length, so as to adjust the brightness of the RGB light;

a detection module, configured to detect brightness variations of the light derived by the light-emergent component to generate an electrical signal detection value;

a determination module, configured to perform an interpolation calculation on the present brightness control signal to obtain an estimated value of the brightness control signal when the electrical signal detection value is less than a preset threshold, and to determine a reaction result in the reagent reaction chamber according to the estimated value of the brightness control signal.

Fig. 1

Fig. 2

31

312

311

332

33

331

Fig. 3

| | |
|---|---|
| S10 | Output a brightness control signal to the light-incident component to control the light-incident component to emit the RGB light, and to adjust the brightness control signal according to a preset step length, so as to adjust the brightness of the RGB light |
| S20 | Detect brightness variations of the light derived by the light-emergent component, so as to generate an electrical signal detection value |
| S30 | When the electrical signal detection value is less than a preset threshold, performing an interpolation calculation on a present brightness control signal to obtain an estimated value of the brightness control signal, and determining a reaction result in the reagent reaction chamber according to the estimated value of the brightness control signal |

Fig. 4

Fig. 5-1

Fig. 5-2

Fig. 6

Fig. 7

The test tube 10

The memory 11 —— The processor 12

Fig. 8

The reagent reaction determination device 100

The brightness control module 101 —— The detection module 102

The determination module 103

Fig. 9

## INTERNATIONAL SEARCH REPORT

| International application No. |
| --- |
| **PCT/CN2021/116782** |

| A. | CLASSIFICATION OF SUBJECT MATTER |
| --- | --- |

G01N 33/48(2006.01)i

According to International Patent Classification (IPC) or to both national classification and IPC

| B. | FIELDS SEARCHED |
| --- | --- |

Minimum documentation searched (classification system followed by classification symbols)

G01N

Documentation searched other than minimum documentation to the extent that such documents are included in the fields searched

Electronic data base consulted during the international search (name of data base and, where practicable, search terms used)

CNPAT, WPI, EPODOC, CNKI, IEEE: 病毒, 阳性, 样品, 检测, 强度, 光, 组织, 核酸, 反应, 亮度, 试样, 电信号, 样本, 强弱, 试剂, virus, positive, sample+, test, light, intensity, bright+, luminance, reagent, react+, optical, measur+, electron+

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 107202903 A (SHENZHEN MINDRAY BIO-MEDICAL ELECTRONICS CO., LTD.) 26 September 2017 (2017-09-26)<br>description, paragraphs [0106]-[0146], and figures 4-6 | 1-12 |
| A | CN 110542662 A (MACCURA MEDICAL ELECTRONICS CO., LTD.) 06 December 2019 (2019-12-06)<br>entire document | 1-12 |
| A | CN 113325425 A (HUNAN YOUZHE TECHNOLOGY CO., LTD.) 31 August 2021 (2021-08-31)<br>entire document | 1-12 |
| A | CN 109856133 A (SHENZHEN XIANGXINGZI TECHNOLOGY CO., LTD.) 07 June 2019 (2019-06-07)<br>entire document | 1-12 |
| A | US 2004152208 A1 (HUTCHINSON, Michael) 05 August 2004 (2004-08-05)<br>entire document | 1-12 |

| ☐ Further documents are listed in the continuation of Box C. | ☑ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **18 May 2022** | **26 May 2022** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

| Patent document cited in search report | | | Publication date (day/month/year) | Patent family member(s) | | | Publication date (day/month/year) |
|---|---|---|---|---|---|---|---|
| CN | 107202903 | A | 26 September 2017 | CN | 111257582 | A | 09 June 2020 |
| | | | | US | 2017315046 | A1 | 02 November 2017 |
| | | | | US | 2019219495 | A1 | 18 July 2019 |
| | | | | US | 2021148809 | A1 | 20 May 2021 |
| | | | | US | 2022099553 | A1 | 31 March 2022 |
| | | | | US | 2020309670 | A1 | 01 October 2020 |
| CN | 110542662 | A | 06 December 2019 | None | | | |
| CN | 113325425 | A | 31 August 2021 | None | | | |
| CN | 109856133 | A | 07 June 2019 | None | | | |
| US | 2004152208 | A1 | 05 August 2004 | CA | 2804073 | A1 | 19 August 2004 |

**INTERNATIONAL SEARCH REPORT**
Information on patent family members

International application No.

**PCT/CN2021/116782**

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2021116782 W